(19) Europäisches Patentamt
European Patent Office
Office européen des brevets

(11) **EP 2 862 508 B1**

(12) **EUROPEAN PATENT SPECIFICATION**

(45) Date of publication and mention
of the grant of the patent:
**09.03.2016 Bulletin 2016/10**

(51) Int Cl.:
**A61B 5/021** (2006.01)       **A61B 5/05** (2006.01)
**A61B 5/00** (2006.01)       *A61B 5/026* (2006.01)

(21) Application number: **14178472.8**

(22) Date of filing: **25.07.2014**

(54) **Sensing system and method for physiology measurements through a measuring signal with overshoot and undershoot pulses**

Erfassungssystem und Verfahren für Physiologiemessungen durch ein Messsignal mit Über- und Unterimpulse

Système et procédé de détection de mesures physiologiques à travers un signal de mesure pulsant avec dépassement d'amplitude positive et négative

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.10.2013   US 201361892174 P
18.07.2014   US 201414335216**

(43) Date of publication of application:
**22.04.2015   Bulletin 2015/17**

(73) Proprietor: **Industrial Technology Research Institute**
**Hsinchu 31040 Taiwan (TW)**

(72) Inventors:
• **Lee, Yen-Hsien**
**Zhongli City, Taoyuan County 32049 (TW)**
• **Lin, Hong-Dun**
**Pingzhen City, Taoyuan County 32449 (TW)**
• **Tseng, Wen-Jen**
**Hsinchu City 30071 (TW)**

(74) Representative: **Gee, Steven William**
**D.W. & S.W. GEE**
**1 South Lynn Gardens**
**London Road**
**Shipston on Stour**
**Warwickshire CV36 4ER (GB)**

(56) References cited:
**EP-A1- 1 710 601       US-A- 5 766 208**
**US-A1- 2008 146 944       US-A1- 2009 128 247**

**Description**

TECHNICAL FIELD

The technical field generally relates to a sensing system and a sensing method for physiology measurements.

BACKGROUND

In current blood pressure measuring devices, auscultation and electron resonance, with a cuff, are widely applied to measure the systolic and diastolic blood pressures of an artery. Therefore, the cuff needs to be inflated and deflated for indirectly measuring non-continuous blood pressure. However, when measuring the continuous blood pressure, the cuff needs to be setup correctly and be inflated and deflated repetitively, which would cause a great inconvenience to the users, and as such, the feasibility and practicality would be significantly less effective.

Sensor systems may be designed for physiology measurement or monitoring or sensing activities of a target such as wrist artery, chest artery, lung activity, and so on.

SUMMARY

The exemplary embodiments of the disclosure may provide a sensing system for physiology measurements and a sensing method thereof.

One exemplary embodiment relates to a sensing system for physiology measurements. The sensing system for physiology measurements may comprise a transmission end including a measuring signal generating module having one or more overshoot and undershoot wave generating circuits with each overshoot and undershoot wave generating circuit generating a measuring signal according to a Pulse Width Modulation (PWM) signal, and a transmitting antenna module having at least one transmitting antenna with each transmitting antenna emitting the measuring signal to a target object, wherein the measuring signal has a characteristics of a pulse wave, and overshoot and undershoot waves are also on the pulse wave; a receiving end having a plurality of receiving antennae with each receiving antenna receiving a reflected signal reflected by the target object; and a plurality of signal analyzing modules included in a plurality of sensors to generate a plurality of object active state signals by analyzing the reflected signal from the each receiving antenna, and transmit the plurality of object active state signals simultaneously or non-simultaneously to a digital signal processing (DSP) device.

Another exemplary embodiment relates to a sensing method for physiology measurements. The sensing method for physiology measurements may comprise: at a transmission end, generating, with each of a plurality of overshoot and undershoot wave generating circuits, a measuring signal according to a Pulse Width Modulation (PWM) signal, and emitting, with each of at least one transmitting antenna, the measuring signal to a target object, wherein the measuring signal has a characteristics of a pulse wave, and overshoot and undershoot waves are also on the pulse wave; at a receiving end, receiving, with each of a plurality of receiving antennae, a reflected signal reflected by the target object; and generating, by a plurality of signal analyzing modules respectively included in a plurality of sensors, a plurality of object active state signals by analyzing the reflected signal from the each receiving antenna, and transmitting the plurality of object active state signals simultaneously or non-simultaneously to a digital signal processing (DSP) device.

BRIEF DESCRIPTION OF THE DRAWINGS

FIG. 1A, FIG. 1B, and FIG. 1C show schematic views of PWM signals PS, measuring signals with pulse overshoot and undershoot, and reflected signals RFS, respectively, according to an exemplary embodiment of the disclosure.

FIG. 2 shows a schematic view of a sensing architecture that applies a multi-system integration, according to one exemplary embodiment of the disclosure.

FIG. 3 shows a sensing system for physiology measurements, according to one exemplary embodiment.

FIG. 4 shows a system structure for the sensing system for physiology measurements, according to an exemplary embodiment.

FIG. 5 shows a schematic view illustrating the measuring signal generating module and the transmitting antenna module are extended to become two sets, according to one exemplary embodiment.

FIG. 6 shows a schematic view illustrating an application scenario of a transmitting antenna sharing by two receiving antennae in a sensing system, according to an exemplary embodiment.

FIG. 7 shows a schematic view illustrating a physical configuration of the sensing system for physiology measurements, according to an exemplary embodiment.

FIG. 8A-FIG. 8D shows four exemplary kinds of an asymmetric comb-shaped antenna module used as the transmitting and receiving antennae, according to exemplary embodiments.

FIG. 9 shows various variations regarding the asymmetric comb-shaped antenna module, according to the exemplary embodiments.

FIIG. 10 shows a schematic view illustrating an application relation between physical circuits of the sensing system and vein, according to an exemplary embodiment.

FIG. 11 shows signals received by sensor 1 and sensor 2 in FIG. 10 after digital sampling, according to an exemplary embodiment.

FIG. 12 shows obtained waveforms being accumulated in the dotted blocks in FIG. 11, according to one exemplary embodiment.

FIG. 13 shows obtained information by accumulating and analyzing the waveforms received by the sensor 1 and the sensor 2 during the diginal signal processing, according to one exemplary embodiment.

FIG. 14 shows a sensing method for physiology measurements, according to one exemplary embodiment.

DETAILED DESCRIPTION OF THE DISCLOSED EMBODIMENTS

Below, exemplary embodiments will be described in detail with reference to accompanying drawings so as to be easily realized by a person having ordinary knowledge in the art. The inventive concept may be embodied in various forms without being limited to the exemplary embodiments set forth herein. Descriptions of well-known parts are omitted for clarity, and like reference numerals refer to like elements throughout.

The exemplary embodiments in the disclosure may provide a sensing technique that may apply a Pulse Width Modulation (PWM) technique to generate a series of measuring signals with pulse overshoot and undershoot, and may apply an asymmetric antenna module to transmit the measuring signals to a target object and receive a plurality of reflected signals reflected by the target object for the physiology measurements. A PWM signal may be, but not limited to a pulse with modulation signal generated by a PWM technique, or implemented by a pulse wave or a square wave with a fixed cycle.

The feature of the PWM is to modulate the transmission power in a time unit. The PWM signals may be applied to generate a series of measuring signals with pulse overshoot and undershoot. For example, when a jitter generator implemented by such as a NAND gate or an AND gate IC is applied, the PWM signals may be transformed into a series of measuring signals with pulse jitter. The asymmetric antenna module may include one or more transmitting antennae and a plurality of receiving antennae. The one or more transmitting antennae may transmit the measuring signals to a target object. The target object may be, but not limited to such as a human body or one of various objects that may reflect the measuring signals. The plurality of reflected signals are the measuring signals reflected by the target object.

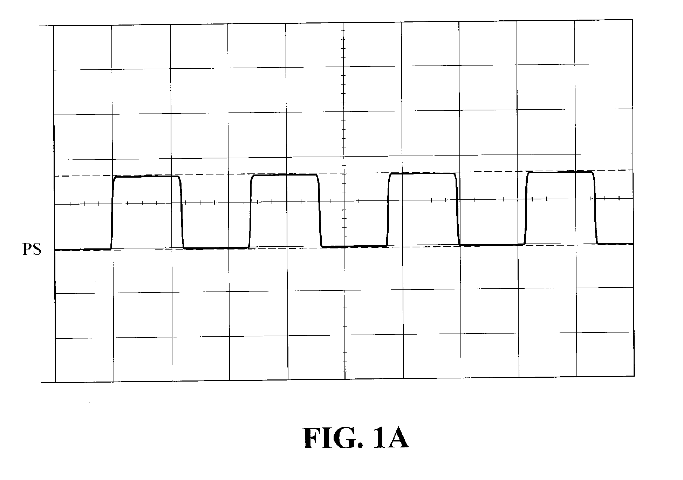

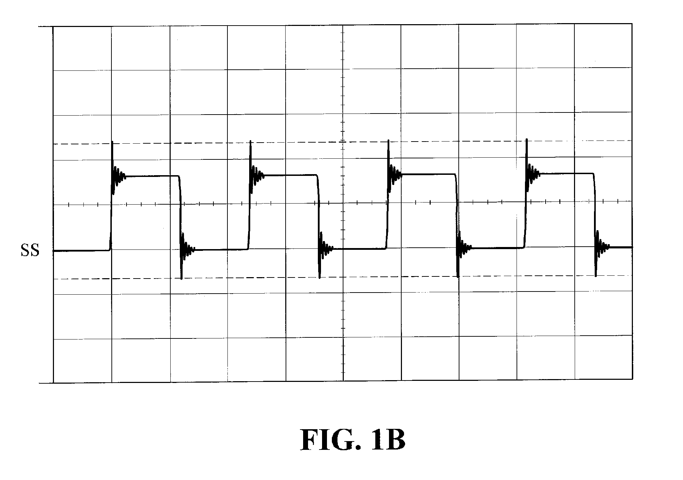

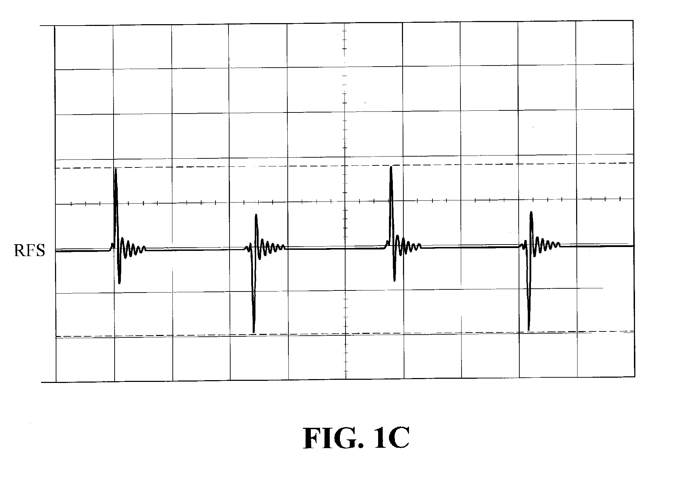

FIG. 1A, FIG. 1B, and FIG. 1C show schematic views of PWM signal PS, measuring signals with overshoot and undershoot waves, and reflected signal RFS, respectively, according to an exemplary embodiment of the disclosure. Referring to FIG. 1A, when a value of the PWM signal PS switches from a high value to a low value or switches from a low value to a high value, the PWM signal PS soon stabilizes because of the characteristics of the PWM technique itself. Referring to FIG. 1B, when a value of the measuring signal SS switches from a high value to a low value or switches from a low value to a high value, the measuring signal SS first undergoes a short shocking period. In this shocking period, the value of the measuring signal SS fluctuates and forms a waveform similar to a shock wave. After this shocking period, the measuring signal SS becomes stable. Referring to FIG. 1C, since the reflected signal RFS is generated by the measuring signal SS with the overshoot and undershoot pulses hitting the target object and then being reflected back, the reflected signal RFS that is generated by reflection also has a similar shocking period. After this shocking period, the reflected signal RFS becomes stable. In this way, the disclosure more accurately senses and judges the active state of the target object by analyzing the reflected signal that also has overshoot and undershoot pulses.

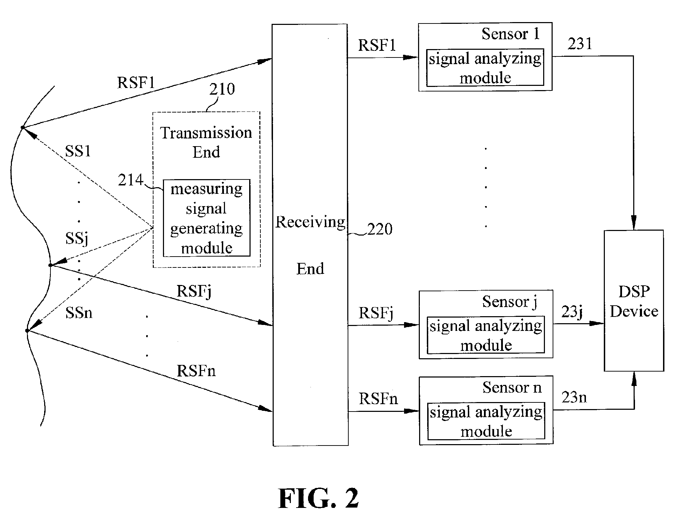

An exemplary architecture of this sensing technique may be implemented by applying a multi-system integration, as shown in FIG. 2. Wherein, the multi-system integration 200 may integrate a transmission end 210 and a receiving end 220, and a plurality of signal analyzing modules included in a plurality of sensors (such as sensor 1 ~ sensor n), respectively. One or more measuring signals (such as SS1~SSn) generated by a measuring signal generating module 214 may be transmitted to a target object (not shown) from the transmission end 210, and one or more measuring signals reflected by the target object (i.e. reflected signals RFS1~RFSn) may be received by the receiving end 220 for further analyzing by the plurality of signal analyzing modules of the plurality of sensors. And then a plurality of object active state signals 231~23n outputted from the plurality of signal analyzing modules are transmitted simultaneously or non-simultaneously to a digital signal processing (DSP) device for subsequently processing.

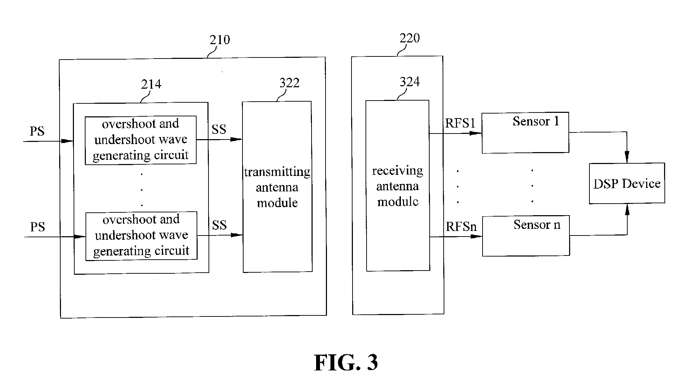

With the exemplary architecture and according an exemplary embodiment, a sensing system for physiology measurements shown in FIG. 3 may comprise the transmission end 210 and the receiving end 220, and a plurality of signal analyzing modules included in a plurality of sensors such as sensor 1~ sensor n. Wherein the transmission end 210 may further include a transmitting antenna module 322, and the measuring signal generating module 214 having one or more overshoot and undershoot wave generating circuits. The measuring signal generating module 214 may generate one or more measuring signals with overshoot and undershoot pulses, wherein each measuring signal SS is generated according to an inputted PWM signal PS. In other words, the measuring signal SS has a characteristics of a pulse wave, and overshoot and undershoot waves are also on the pulse wave. Each of the overshoot and undershoot wave generating circuits included in the measuring signal generating module 214 may generate a measuring signal SS. The transmitting antenna module 322 may include at least one transmitting antenna TX. Each transmitting antenna may transmit a measuring signal SS to a target object. The receiving end 220 may include a receiving antenna module 324 having a plurality of receiving antennae. Each of the plurality of receiving antennae may receive a RFS signal (a measuring signal reflected by the target object) for further analyzing by a signal analyzing module included in an associated sensor. After the analyzing, each object active state signal BS outputted from each signal analyzing module may be obtained, and then a plurality of object active state signals outputted from the plurality of signal analyzing modules are transmitted simultaneously or non-simultaneously to a DSP device for subsequently processing.

When the measuring signal SS is continuously transmitted by the transmitting antenna module 322 in the form of radiation, the reflected signal RFS is continuously reflected back once the measuring signal SS hits the target object, and then the reflected signal RFS is continuously received by the receiving antenna module 324. When an active state or a movement state of the target object changes, an angle and/or a hitting position at which the measuring signal SS hits the target object also changes, thereby resulting in a change in a frequency, a waveform or a receiving time of the reflected signal RFS received by the receiving antenna 324. In other words, according to the exemplary embodiments, real-time active state information of the target object may be effectively obtained by analyzing these reflected signals RFS.

In an application exemplar, the transmitting antenna module may be implemented by one transmitting antenna. In the application scenario, a plurality of reflected signals received by the plurality of receiving antennae may be a plurality of measuring signals that are transmitted, by the one transmitting antenna, to the target object and reflected by the target object. In another application exemplar, the transmitting antenna module may be implemented by a plurality of transmitting antennae, and a plurality of reflected signals received by the plurality of receiving antennae may be a plurality of measuring signals that are transmitted respectively by the plurality of transmitting antennae to the target object and reflected by the target object.

In an application exemplar, the measuring signal generating module 214 may be implemented by a plurality of overshoot and undershoot wave generating circuits with the each overshoot and undershoot wave generating circuit being coupled to an associated signal analyzing module of the plurality of sensors for generating the measuring signal used by the associated sensor, respectively. In another application exemplar, the measuring signal generating module may be implemented by one overshoot and undershoot wave generating circuit coupled to the plurality of signal analyzing modules for generating a plurality of measuring signals according to a series of inputted PWM signals, and then the transmitting antenna module transmits the plurality of measuring signals to the target object.

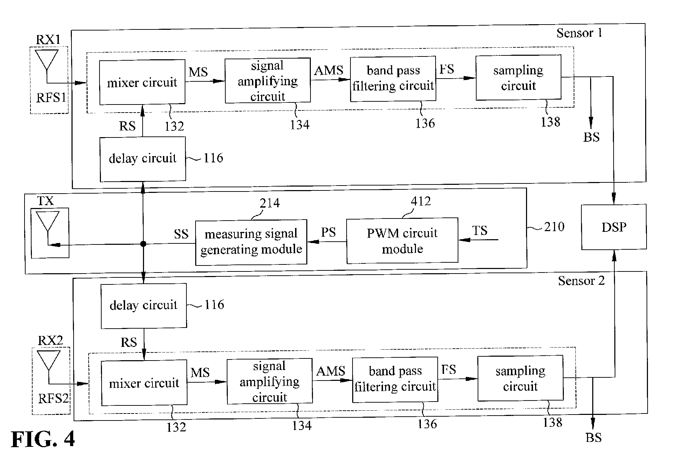

Take two sensors (referred as Sensor 1 and Sensor 2) as an exemplar. FIG. 4 shows a system structure for the sensing system for physiology measurements, according to an exemplary embodiment. As shown in FIG. 4, the transmission end 210 may further include a PWM circuit module 412 coupled to the measuring signal generating module 214 to generate the PWM signal PS according to a clock signal TS, thereby providing the PWM signal PS to the measuring signal generating module 214. At the receiving end 220, each sensor may receive a reflected signal RFS from a receiving antenna (referred as RX1 and RX2 for Sensor 1 and Sensor 2, respectively), and include a delay circuit 116 and a signal analyzing module. The signal analyzing module may further include a mixer circuit 132, a signal amplifying circuit 134, a band pass filtering circuit 136, and a sampling circuit 138. The delay circuit 116 is coupled to the measuring signal generating module 214 to generate a reference signal RS according to the measuring signal SS. The mixer circuit 132 is coupled to the receiving antenna module 324 and the delay circuit 116 to mix the reflected signal RFS and the reference signal RS to be a mixing signal MS. The signal amplifying circuit 134 is coupled to the mixer circuit 132 to amplify the mixing signal MS to be an amplified mixing signal AMS. The band pass filtering circuit 136 is coupled to the signal amplifying circuit 134 to perform a filtering operation on the amplified mixing signal AMS to generate a filtered signal FS. The sampling circuit 138 is coupled to the band pass filtering circuit 136 to perform a sampling operation on the filtered signal FS, thereby obtaining the object active state signal BS. The signal amplifying circuit 134, the band pass filtering circuit 136 and the sampling circuit 138 may all be adjustable according to practical or design requirements. These circuits are not the focus of the disclosure, and the detailed descriptions thereof are omitted herein.

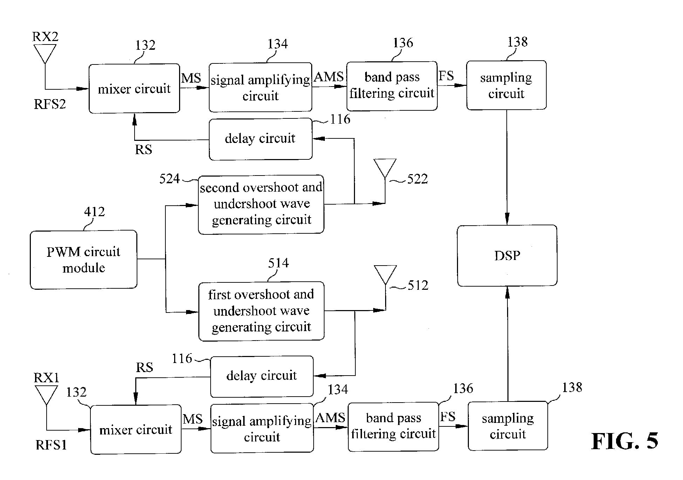

According to an exemplary embodiment, the measuring signal generating module 214 and the transmitting antenna module 322 may be extended to become n sets, with each set including an overshoot and undershoot wave generating circuit and a transmitting antenna and being provided to each sensor of the sensor 1~sensor n, respectively, for use. Take two sensors, such as sensor 1 and sensor 2, as an exemplar. As shown in FIG. 5, the measuring signal generating module 214 may include a first overshoot and undershoot wave generating circuit 514 and a second overshoot and undershoot wave generating circuit 524. The transmitting antenna module 322 may include a first transmitting antenna 512 and a second transmitting antenna 522. A first set formed by the first overshoot and undershoot wave generating circuit 514 and the first transmitting antenna 512 may be used by the sensor 1, and a second set formed by the second overshoot and undershoot wave generating circuit 524 and the second transmitting antenna 522 may be used by the sensor 2, respectively, for use. In other words, each sensor uses respective overshoot and undershoot wave generating circuit and respective transmitting antenna, according to the exemplary embodiment. In this exemplar, the sensor 1 and the sensor 2 share with the PWM circuit module 412 that provides the inputted PWM signals to both of the first overshoot and undershoot wave generating circuit 514 and the second overshoot and undershoot wave generating circuit 524.

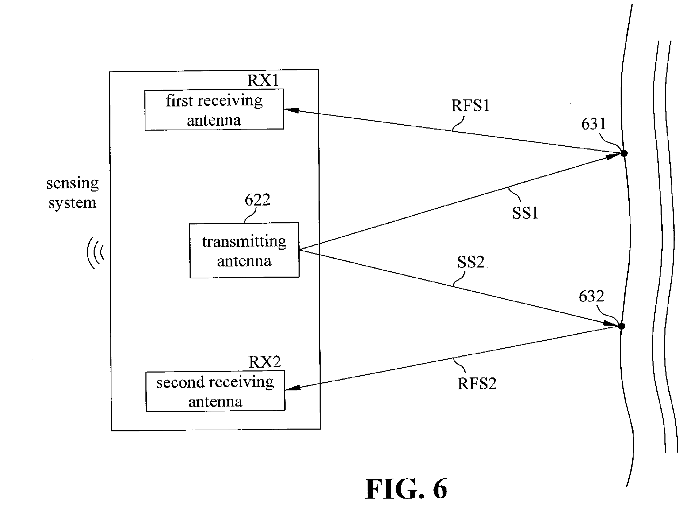

According to another exemplary embodiment, the plurality of sensors may share with the transmitting antennae in the transmitting antenna module 322. FIG. 6 shows a schematic view illustrating an application scenario of a transmitting antenna sharing by two receiving antennae in a sensing system, according to an exemplary embodiment. Referring to the exemplar in FIG. 6, in the sensing system, there are two receiving antennae referred as a first receiving antenna RX1 and a second receiving antenna RX2 at the receiving end, and one transmitting antenna 622 at the transmission end. When the measuring signal SS is continuously transmitted by the transmitting antenna 622 in the form of radiation, the reflected signal RFS is continuously reflected back once the measuring signal SS hits a measuring point of a target object (not shown), and then the reflected signal RFS is continuously received by one of the two receiving antennae. For example, when a first measuring signal SS1 is transmitted by the transmitting antenna 622, a first reflected signal RFS1 is reflected back once the measuring signal SS1 hits a first measuring point 631 of the target object, and then the first reflected signal RFS1 is received by the first receiving antenna RX1. Similarly, when a second measuring signal SS2 is transmitted by the transmitting antenna 622, a second reflected signal RFS2 is reflected back once the measuring signal SS2 hits a second measuring point 632 of the target object, and then the second reflected signal RFS2 is received by the second receiving antenna RX2. The first receiving antenna and the second antenna could be implemented by more than one antennae.

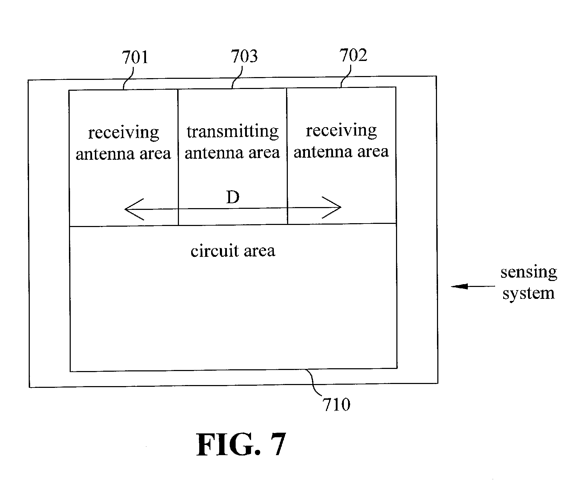

FIG. 7 shows a schematic view illustrating a physical configuration of the sensing system for physiology measurements, according to an exemplary embodiment. Referring to FIG. 7, the sensing system may be configured into a circuit area 710, and a plurality of antenna areas, such as two receiving antenna areas 701 and 702 and a transmitting antenna area 703. The measuring signal generating module 214 and the plurality of signal analyzing modules may be configured in the circuit area 710. The plurality of receiving antennae may be distributed in a plurality of receiving antenna areas surrounding a transmitting antenna area. Take two sensors as an exemplar. Two receiving antennae may be configured in two receiving antenna areas 701 and 702, respectively. The transmitting antenna module 322 may be configured between the receiving antenna areas 701 and 702, where D is a distance between the two receiving antennae. In other words, the sensing system for physiology measurements may be further configured into a circuit area containing the measuring signal generating module 214 and the plurality of signal analyzing modules, and a plurality of antenna areas containing the plurality of receiving antennae and the transmitting antenna module 322 surrounded by the plurality of receiving antennae.

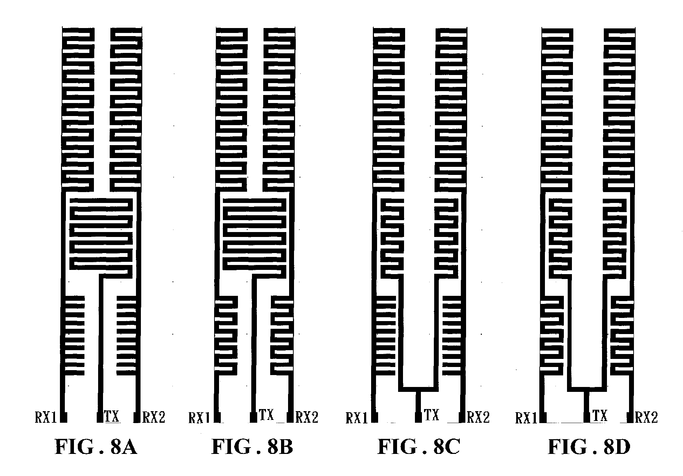

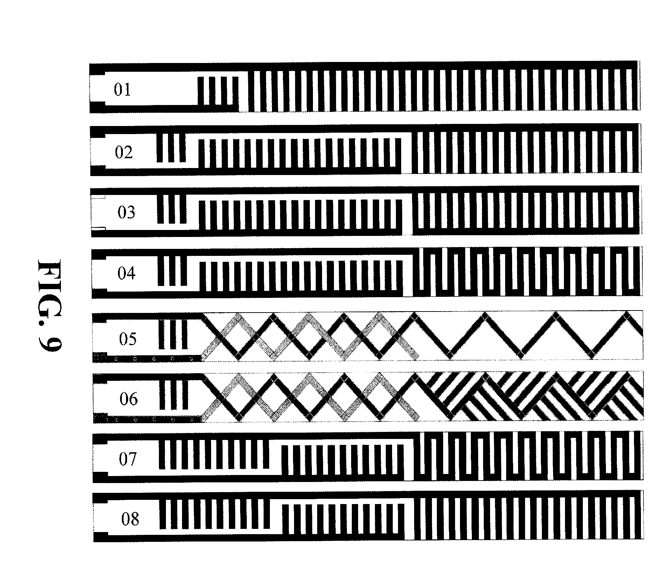

The following describes the antenna design. For the transmitting antenna in the transmitting antenna module and the receiving antennae at the receiving end, an asymmetric comb-shaped antenna module may be used as the transmitting and receiving antennae. FIG. 8A-FIG. 8D shows four exemplary types of an asymmetric comb-shaped antenna module used as the transmitting and receiving antennae, according to exemplary embodiments. For each exemplary asymmetric comb-shaped antenna module, assume that there are two receiving antennae at the receiving end, and one transmitting antenna in the transmitting antenna module. FIG. 9 further shows various variations regarding the asymmetric comb-shaped antenna module, according to the exemplary embodiments. According to the exemplary embodiments in the disclosure, any antenna combinations of these various variations regarding the asymmetric comb-shaped antenna module shown in FIG. 9 may be used as the transmitting and receiving antennae. In other words, the at least one transmitting antenna of the transmitting antenna module and the plurality of receiving antennae at the receiving end may respectively have a comb-shaped structure.

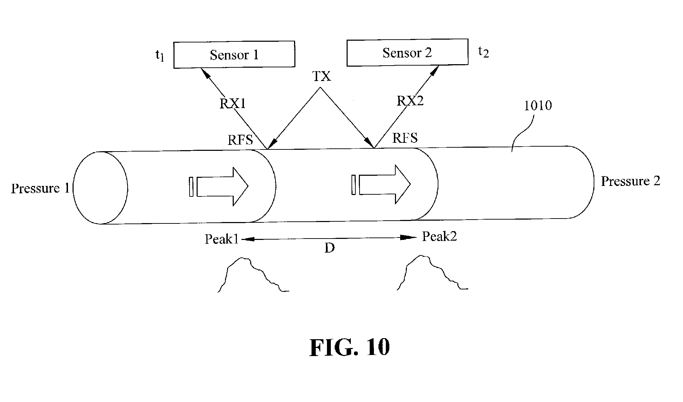

According to the exemplary embodiments, the sensing system may be applied to measure pressures of veins such as but not be limited to the systolic and diastolic blood pressures of an artery. FIG. 10 shows a schematic view illustrating an application relation between physical circuits of the sensing system and vein of a target object such as a human body, according to an exemplary embodiment. In the exemplary embodiment of FIG. 10, the physical circuits of the sensing system may use two sensors such as sensor 1 and sensor 2, and two receiving antennae such as RX1 and RX2 and at least one transmitting antenna such as TX to measure pressure 1 and pressure 2 of vein 1010 such as the systolic and diastolic blood pressures of the artery. The measuring signal generating module of the sensing system may generate a first measuring signal and a second measuring signal according to an inputted Pulse Width Modulation (PWM) signal. The at least one transmitting antenna TX transmits the first and the second measuring signals to the vein 1010 for being used by the sensor 1 and the sensor 2, respectively. Each of the two receiving antennae RX1 and RX2 may respectively receive a reflected signal RFS reflected by the vein 1010 for being used and analyzed by the sensor 1 and the sensor 2, respectively. While a time difference $t_2$ is larger than a time difference $t_1$ (i.e. receiving time of a first scattered pulse signal - emitting time of a first radiated pulse signal), a pulse peak would occur, wherein the time difference $t_1$ is a previous time difference to the time difference $t_2$, and Peak 1 corresponds to the pulse peak received by the RX1 for sensor 1, Peak 2 corresponds to the pulse peak received by the RX2 for sensor 2.

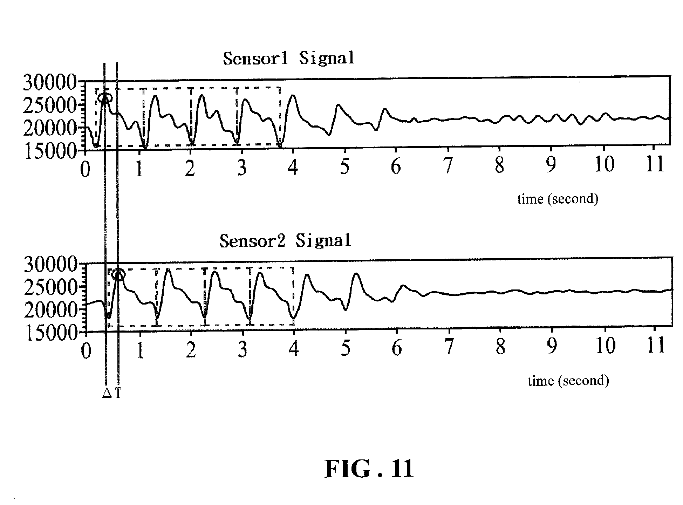

FIG. 11 further shows signals received by sensor 1 and sensor 2 after digital sampling, according to an exemplary embodiment. Referring to FIG. 11, as may be seen, a time lapse $\Delta T$, i.e. a pulse time difference of the first pulse peak and the second pulse peak, exists between the signals received by sensor 1 and sensor 2 because the pulse experiences lagging during transmission. In other words, the time lapse $\Delta T$ may be obtained through two different generating times of two different pulse peaks received by two different antennae, as shown in the following formula for the case of FIG. 11:

$$\Delta T = \text{generating time of the second pulse peak} - \text{generating time of the first pulse peak.}$$

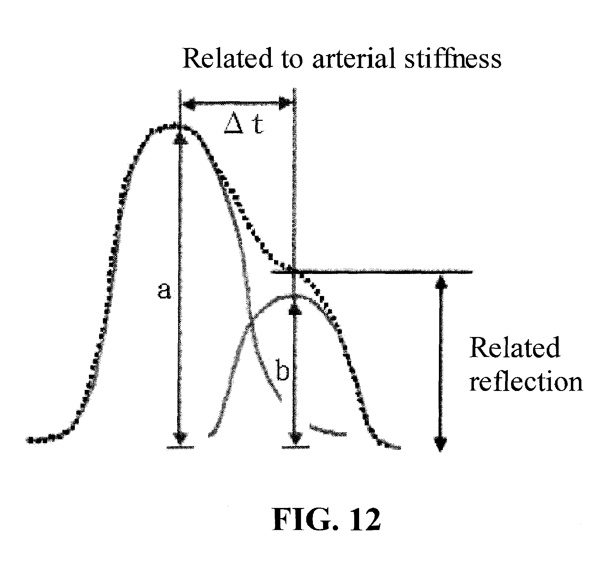

The following uses blood pressure measurement as an exemplar to describe the computation of the systolic and diastolic blood pressures. In processing signal, the receiving time $t_1$ and $t_2$ of the pulses are used to calculate the pulse wave velocity (PWV). For example, the PWV may be expressed as a function of a distance D between two different receiving antennae, and a time difference of two signals received by different sensors, respectively. One of examples is, PWV =D/$\Delta T$. In other words, the time lapse $\Delta T$ may be used as the time difference. In the DSP, the waveform in the dotted blocks shown in FIG. 11 may be accumulated to obtain the waveform in the FIG. 12. According to the waveform transmission and reflection theory, the pressure sensing waveform obtained by a wrist may be divided into two parts, wherein the first part of the waveform is the pulse reaching the wrist by following the path of a main artery (i.e. the waveform with a height a in the FIG. 12); and the second part of the waveform is the pulse reaching the micro-vascular of a hand and then reflected (i.e. the waveform with a height b in the FIG. 12) back to the wrist.

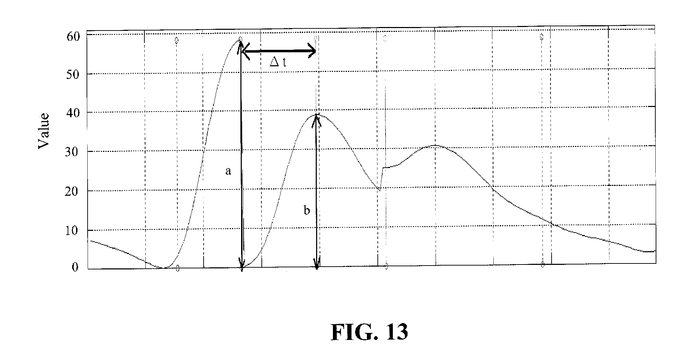

The time lapse $\Delta t$ between the main wave and the reflected wave (vascular stiffness index SI= $\Delta t$) and the height ratio of the main wave and the reflected wave (vascular reflection index RI=a/b) may be used to compute the extent of hardening of the artery, and may also be used to detect the blood pressure. Based on the above method, according to an exemplary embodiment, the waveforms received by the sensor 1 and the sensor 2 are accumulated and analyzed during the digital signal processing, and then the information shown in FIG. 13 is obtained. In other words, applying the waveform transmission and reflection theory such as Bernoulli's theory, a linear relation between the blood pressure (BP) and pulse wave velocity (PWV) may be expressed as

$$BP = a \times PWV + b$$

wherein a and b are vascular parameters calculated by the original pulse signal measured by a sensor, and the pulse wave velocity (PWV) is a measure of a first measure point and a second measure point of a target object such as an artery; a and b are vascular parameters calculated by the original pulse signal measured by the sensor.

Through further analysis on the measured wave and vascular parameter analysis, the relation among the vascular stiffness index (SI), vascular reflection index (RI) and heart rate (HR) may be computed, and the blood pressure (BP) is a function of the pulse wave velocity (PWV), vascular stiffness index (SI), vascular reflection index (RI) and heart rate (HR), i.e. BP = F(PWV, SI, RI, HR). Therefore, the aforementioned method may detect the blood pressure such as the systolic and diastolic blood pressures, through combining the sensing system of the disclosure and the blood flow velocity algorithm. And, the systolic blood pressure $BP_{Sys}$ and the diastolic blood pressure $BP_{Dia}$ of an artery may be obtained by using the algorithm including the following formula.

$$BP_{Sys} = a_1 \times PWV + b_1$$

$$BP_{Dia} = a_2 \times PWV + b_2$$

wherein the pulse wave velocity (PWV) may be calculated by the formula PWV $= D/\Delta T$, $a_1$ and $b_1$ are vascular parameters calculated by the original pulse signal measured by the sensor 1, and $a_2$ and by are vascular parameters calculated by the original pulse signal measured by the sensor 2. Therefore, this may improve the precision of the transformation from the PWV to the pressure measurements, and the feasibility and practicality will be significantly effective to the users.

If the regression analysis and comparison are performed on the blood pressure measurements obtained by the standard blood pressure meter with a cuff, obtained blood pressure equations are:

$$\text{systolic blood pressure SBP} = 0.00022318X^2 + 0.21107X + 90.381,$$

$$\text{diastolic blood pressure DBP} = 0.00044459X^2 + 0.29295X + 38.33$$

And, the cuff needs to be inflated and deflated for indirectly measuring non-continuous blood pressure. When measuring continuous blood pressure, the cuff needs to be setup correctly and be inflated and deflated repetitively. In one exemplary embodiment,

$$X = \frac{C}{\left[ \dfrac{SI}{PWV} \times \dfrac{1}{\Delta T} \times \ln\left( \sqrt{\dfrac{HR \times SI}{RI}} \right) \right]}$$

wherein C is a constant.

Accordingly, the sensing system may also be applied to measure pressures, such as blood pressure measurement of the main arteries of the chest, blood pressure measurement of the carotid arteries of the neck (also applicable to brain pressure measurement), blood pressure measurement of the peripheral vascular, and so on. The sensing system may further comprise a signal processing device including a wireless module, and a microcontroller to calculate pressures. The microcontroller may have a calculation unit which has an algorithm such as the aforementioned blood flow velocity algorithm. The signal processing device may use the wireless module to communicate with the plurality of sensors via a wireless protocol. The wireless protocol may be, but not limited to a Bluetooth protocol. The calculation unit may uses the algorithm to calculate the pressures such as the aforementioned blood pressures. The algorithm may include the aforementioned relation formula between the blood pressure (BP) and pulse wave velocity (PWV).

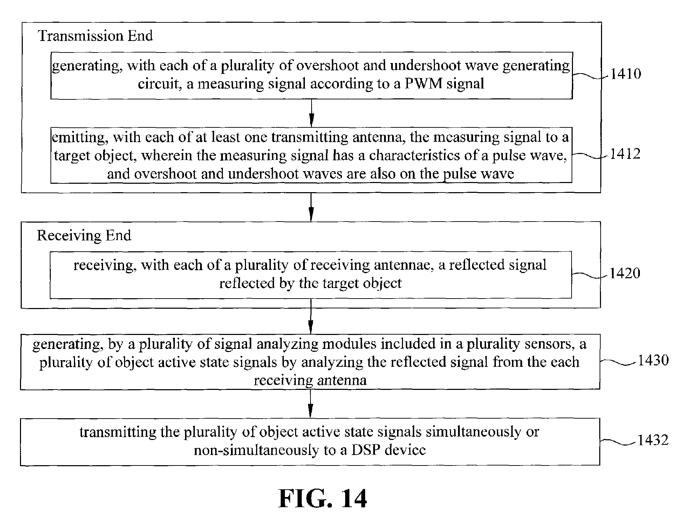

FIG. 14 shows a sensing method for physiology measurements, according to one exemplary embodiment. Referring to FIG. 14, the sensing method for physiology measurements may comprise: at a transmission end, generating, with each of a plurality of overshoot and undershoot wave generating circuit, a measuring signal according to a Width Modulation (PWM) signal (step 1410), and emitting, with each of at least one transmitting antenna, the measuring signal to a target object, wherein the measuring signal has a characteristics of a pulse wave, and overshoot and undershoot waves are also on the pulse wave (step 1412); at a receiving end, receiving, with each of a plurality of receiving antennae, a reflected signal reflected by the target object (step 1420); and generating, by a plurality of signal analyzing modules respectively included in a plurality sensors, a plurality of object active state signals by analyzing the reflected signal from the each receiving antenna (step 1430), and transmitting the plurality of object active state signals simultaneously or non-simultaneously to a digital signal processing (DSP) device (step 1432).

As aforementioned, the measuring signal may be generated by generating the pulse width modulation signal according to a clock signal, and modulating the pulse width modulation signal to be the measuring signal with the overshoot and undershoot pulses by means of a digital signal processing. As shown in the examples of FIG. 8A-FIG. 8D and FIG. 9, an asymmetric comb-shaped antenna module, or various variations regarding the asymmetric comb-shaped antennae module may be used as the at least one transmitting antenna at the transmission end and the plurality of receiving antennae at the receiving end. The detailed descriptions thereof are omitted herein.

The sensing method may also be applied to measure pressures of the target object, such as but not be limited to the systolic blood pressure $BP_{Sys}$ and the diastolic blood pressure $BP_{Dia}$ of an artery, as described in FIG. 10. In the digital signal processing, the calculation of a pressure measurement of the target object may include: calculating a pulse time difference of a first pulse peak and a second pulse peak, wherein the first pulse peak and the second pulse peak are received by two different antennae of the plurality of antennae; calculating a pulse wave velocity (PWV) by using the pulse time difference and a distance between the first pulse peak and the second pulse peak; calculating each pressure of one or more pressures, by expressing a linear relation between the pressure and the pulse wave velocity. As shown in the aforementioned examples and descriptions, the pressure measurement may be chosen from one or more combinations of a blood pressure measurement of a plurality of main arteries of a chest, a blood pressure measurement of a plurality of carotid arteries of a neck, a brain pressure measurement, a blood pressure measurement of a peripheral vascular. The detailed descriptions thereof are omitted herein.

According to another exemplary embodiment of the present disclosure, there is provided a chip for physiology measurements. The chip for physiology measurements may integrate at least one transmitting antenna, a plurality of receiving antennae, and an integrated circuit. The integrated circuit may further include one or more overshoot and undershoot wave generating circuits and a plurality of sensors. Each overshoot and undershoot wave generating circuit and each circuit in each sensor are configured to perform their associated acts described in the above embodiments. An exemplary physical configuration of the chip for physiology measurements may be configured into a circuit area and a plurality of antenna areas, similar to that of the FIG. 7. In other words, the sensing system for physiology measurements may be implemented by a chip, and the chip may integrate at least one transmitting antenna, a plurality of receiving antennae, and an integrated circuit having one or more overshoot and undershoot wave generating circuits and a plurality of sensors.

The exemplary embodiments have at least the following features: (1) The sensing technique for physiology measurements, such as for blood pressure, may be realized with PWM, overshoot and downshoot pulses, and an asymmetric comb-shaped antenna module or various variations thereof; (2) the outputs, such as the plurality of object active state signals from the sensors are transmitted simultaneously or non-simultaneously to a digital signal processor for further processing and/or analyzing; (3) the measuring signal generating module and the transmitting antenna module may be extended to become n sets, with each set including an overshoot and undershoot wave generating circuit and a transmitting antenna and being provided to each sensor of the n sensors, respectively; and (4) each pressure of one or more pressures may be calculated by expressing a linear relation between the pressure and a pulse wave velocity.

It will be apparent to those skilled in the art that various modifications and variations can be made to the disclosed embodiments. It is intended that the specification and examples be considered as exemplary only, with a true scope of the disclosure being indicated by the following claims and their equivalents.

## Claims

1.  A sensing system for physiology measurements, comprising:

     a transmission end (210) including a measuring signal generating module (214) having one or more overshoot and undershoot wave generating circuits with each overshoot and undershoot wave generating circuit generating a measuring signal (SS) according to a Pulse Width Modulation (PWM) signal (PS), and a transmitting antenna module (322) having at least one transmitting antenna with each transmitting antenna emitting the measuring signal to a target object, wherein the measuring signal (SS) has a characteristics of a pulse wave, and overshoot and undershoot waves are also on the pulse wave;
     a receiving end (220) having a plurality of receiving antennae with each receiving antenna receiving a reflected signal (RFS) reflected by the target object; and
     a plurality of signal analyzing modules included in a plurality of sensors to generate a plurality of object active state signals (231~23n) by analyzing the reflected signal (RFS) from the each receiving antenna, then transmit the plurality of object active state signals (231~23n) simultaneously or non-simultaneously to a digital signal processor.

2.  The sensing system as claimed in claim 1, wherein the transmitting antenna module (322) is implemented by one transmitting antenna, and a plurality of reflected signals (RFS1~RFSn) received by the plurality of receiving antennae are a plurality of measuring signals that are transmitted by said one transmitting antenna to the target object and reflected by the target object.

3.  The sensing system as claimed in claim 1, wherein the transmitting antenna module (322) is implemented by a plurality of transmitting antennae, and a plurality of reflected signals (RFS1~RFSn) received by the plurality of receiving antennae are a plurality of measuring signals (SS1~SSn) that are transmitted respectively by said plurality of transmitting antennae to the target object and reflected by the target object.

4.    The sensing system as claimed in claim 1, wherein the measuring signal generating module (214) is implemented by a plurality of overshoot and undershoot wave generating circuits with the each overshoot and undershoot wave generating circuit being coupled to an associated sensor of the plurality of sensors for generating the measuring signal (SS) used by the associated sensor, respectively.

5.    The sensing system as claimed in claim 1, wherein the measuring signal generating module (214) is implemented by one overshoot and undershoot wave generating circuit coupled to the plurality of sensors for generating a plurality of measuring signals according to a series of inputted PWM signals, and then the transmitting antenna module (322) transmits the plurality of measuring signals to the target object.

6.    The sensing system as claimed in claim 1, wherein the transmission end (210) further include a PWM circuit module (412) coupled to the measuring signal generating module (214) to generate the PWM signal (PS) according to a clock signal (TS).

7.    The sensing system as claimed in claim 1, wherein the sensing system is further configured into a circuit area (710) containing the measuring signal generating module (214) and the plurality of sensors, and a plurality of antenna areas containing the plurality of receiving antennae and the transmitting antenna module (322) surrounded by the plurality of receiving antennae.

8.    The sensing system as claimed in claim 1, wherein for the at least one transmitting antenna of the transmitting antenna module (322) and the plurality of receiving antennae at the receiving end (220) respectively have a comb-shaped structure.

9.    The sensing system as claimed in claim 1, wherein each sensor of the plurality sensors further includes a delay circuit (116) and a signal analyzing module of the plurality of signal analyzing modules, and the delay circuit (116) generates a reference signal (RS) according to the measuring signal (SS).

10.   The sensing system as claimed in claim 9, wherein the signal analyzing module included in the sensor further includes:

        a mixer circuit (132) coupled to the delay circuit (116) and one of the plurality of receiving antenna, and mixing the reflected signal (RFS) and the reference signal (RS) to be a mixing signal (MS);
        a signal amplifying circuit (134) coupled to the mixer circuit (132), and amplifying the mixing signal (MS) to be an amplified mixing signal (AMS);
        a band pass filtering circuit (136) coupled to the signal amplifying circuit (134) and performing a filtering operation on the amplified mixing signal (AMS) to generate a filtered signal (FS); and
        a sampling circuit (138) coupled to the band pass filtering circuit (136) and performing a sampling operation on the filtered signal (FS) to obtain an object active state signal.

11.   The sensing system as claimed in claim 1, wherein the sensing system is implemented by a chip, and the chip integrates the at least one transmitting antenna, the plurality of receiving antennae, and an integrated circuit having the one or more overshoot and undershoot wave generating circuits and the plurality of sensors.

12.   The sensing system as claimed in claim 1, wherein the sensing system further includes a signal processing device, and the signal processing device further includes:

        a wireless module that communicates with the plurality of sensors; and
        a microcontroller that applies a calculation unit to calculate one or more pressures of the target object.

13.   The sensing system as claimed in claim 12, wherein the calculation unit uses an algorithm to calculate one or more blood pressures, and the algorithm includes a relation formula between the one or more pressures and a pulse wave velocity (PWV), and the PWV is a measure for two measure points (631, 632) of the target object.

14.   The sensing system as claimed in claim 13, wherein the PWV is expressed as a function of a distance (D) between two different receiving antennae (RX1, RX2) of the plurality of antennae, and a time difference ($\Delta$T) of two signals received by two different sensors of the plurality of sensors, respectively.

15.   The sensing system as claimed in claim 14, wherein the time difference ($\Delta$T) is a time lapse obtained through two different generating times of two different pulse peaks received by the two different antennae (RX1, RX2).

**16.** A sensing method for physiology measurements, comprising:

at a transmission end (210), generating, with each of a plurality of overshoot and undershoot wave generating circuits, a measuring signal (SS) according to a Pulse Width Modulation (PWM) signal (PS), and emitting, with each of at least one transmitting antenna, the measuring signal (SS) to a target object, wherein the measuring signal (SS) has a characteristics of a pulse wave, and overshoot and undershoot waves are also on the pulse wave;

at a receiving end (220), receiving, with each of a plurality of receiving antennae, a reflected signal (RFS) reflected by the target object;

generating, by a plurality of signal analyzing modules respectively included in a plurality of sensors, a plurality of object active state signals (231~23n) by analyzing the reflected signal (RFS) from the each receiving antenna; and

transmitting the plurality of object active state signals (231~23n) simultaneously or non-simultaneously to a digital signal processing device.

**17.** The sensing method as claimed in claim 16, wherein the measuring signal (SS) is generated by generating the PWM signal (PS) according to a clock signal (TS), and modulating the PWM signal (PS) to be the measuring signal (SS) with the overshoot and undershoot pulses by means of a digital signal processing.

**18.** The sensing method as claimed in claim 16, wherein an asymmetric comb-shaped antenna module, or various variations regarding the asymmetric comb-shaped antenna module is used as at least one transmitting antenna of a transmitting antenna module (322) and the plurality of receiving antennae at the receiving end (220).

**19.** The sensing method as claimed in claim 16, the sensing method further performs a calculation of a pressure measurement of the target object, and the calculation of the pressure measurement includes:

calculating a pulse time difference of a first pulse peak and a second pulse peak, wherein the first pulse peak and the second pulse peak are received by two different receiving antennae (RX1, RX2) of the plurality of receiving antennae;

calculating a pulse wave velocity (PWV) by using the pulse time difference and a distance (D) between the first pulse peak and the second pulse peak; and

calculating each pressure of one or more pressures, by expressing a linear relation between the pressure and the pulse wave velocity.

**20.** The sensing method as claimed in claim 19, wherein the pressure measurement is chosen from one or more combinations of a blood pressure measurement of a plurality of main arteries of a chest, a blood pressure measurement of a plurality of carotid arteries of a neck, a brain pressure measurement, a blood pressure measurement of a peripheral vascular.

**Patentansprüche**

**1.** Erfassungssystem für physiologische Messungen, umfassend:

eine Sendeseite (210), enthaltend ein Messsignalgenerierungsmodul (214) mit einer oder mehreren Überschwingungs- und Unterschwingungswellen generierenden Schaltungen, wobei jede Überschwingungs- und Unterschwingungswellen generierende Schaltung ein Messsignal (SS) gemäß einem PWM-Signal (Pulse Width Modulation, Pulsweitenmodulation) (PS) generiert, und ein Sendeantennenmodul (322) mit mindestens einer Sendeantenne, wobei jede Sendeantenne das Messsignal zu einem Zielobjekt emittiert, wobei das Messsignal (SS) ein Merkmal einer Pulswelle hat und Überschwingungs- und Unterschwingungswellen ebenfalls auf der Pulswelle sind;

eine Empfangsseite (220) mit einer Mehrzahl von Empfangsantennen, wobei jede Empfangsantenne ein reflektiertes Signal (RFS) empfängt, das durch das Zielobjekt reflektiert wurde; und

eine Mehrzahl von Signalanalysemodulen, die in einer Mehrzahl von Sensoren enthalten sind, um eine Mehrzahl von Objektaktivstatussignalen (231~23n) zu generieren, indem das reflektierte Signal (RFS) von jeder Empfangsantenne analysiert wird, dann die Mehrzahl von Objektaktivstatussignalen (231~23n) gleichzeitig oder ungleichzeitig zu einem digitalen Signalprozessor gesendet werden.

2. Erfassungssystem gemäß Anspruch 1, wobei das Sendeantennenmodul (322) durch eine Sendeantenne implementiert ist, und eine Mehrzahl von reflektierten Signalen (RFS1~RFSn), die durch die Mehrzahl von Empfangsantennen empfangen werden, eine Mehrzahl von Messsignalen sind, die durch die eine Sendeantenne zu dem Zielobjekt gesendet werden und durch das Zielobjekt reflektiert werden.

3. Erfassungssystem gemäß Anspruch 1, wobei das Sendeantennenmodul (322) durch eine Mehrzahl von Sendeantennen implementiert ist, und eine Mehrzahl von reflektierten Signalen (RFS1~RFSn), die durch die Mehrzahl von Empfangsantennen empfangen werden, eine Mehrzahl von Messsignalen (SS1~SSn) sind, die jeweils durch die Mehrzahl von Sendeantennen zu dem Zielobjekt gesendet werden und durch das Zielobjekt reflektiert werden.

4. Erfassungssystem gemäß Anspruch 1, wobei das Messsignalgenerierungsmodul (214) durch eine Mehrzahl von Überschwingungs- und Unterschwingungswellen generierenden Schaltungen implementiert ist, wobei jede Überschwingungs- und Unterschwingungswellen generierende Schaltung mit einem zugeordneten Sensor aus der Mehrzahl von Sensoren gekoppelt ist, um das Messsignal (SS) zu generieren, das vom jeweiligen zugeordneten Sensor verwendet wird.

5. Erfassungssystem gemäß Anspruch 1, wobei das Messsignalgenerierungsmodul (214) durch eine Überschwingungs- und Unterschwingungswellen generierende Schaltung implementiert ist, die mit der Mehrzahl von Sensoren gekoppelt ist, um eine Mehrzahl von Messsignalen gemäß einer Serie von eingegebenen PWM-Signalen zu generieren, und dann das Sendeantennenmodul (322) die Mehrzahl von Messsignalen zu dem Zielobjekt sendet.

6. Erfassungssystem gemäß Anspruch 1, wobei die Sendeseite (210) des Weiteren ein PWM-Schaltungsmodul (412) enthält, das mit dem Messsignalgenerierungsmodul (214) gekoppelt ist, um das PWM-Signal (PS) gemäß einem Taktsignal (TS) zu generieren.

7. Erfassungssystem gemäß Anspruch 1, wobei das Erfassungssystem des Weiteren in einen Schaltungsbereich (710) integriert ist, der das Messsignalgenerierungsmodul (214) und die Mehrzahl von Sensoren enthält, und eine Mehrzahl von Antennenbereichen die Mehrzahl von Empfangsantennen enthält und das Sendeantennenmodul (322) von der Mehrzahl von Empfangsantennen umgeben ist.

8. Erfassungssystem gemäß Anspruch 1, wobei die mindestens eine Sendeantenne des Sendeantennenmoduls (322) und die Mehrzahl von Empfangsantennen an der Empfangsseite (220) jeweils eine kammförmige Struktur haben.

9. Erfassungssystem gemäß Anspruch 1, wobei jeder Sensor aus der Mehrzahl von Sensoren des Weiteren eine Verzögerungsschaltung (116) und ein Signalanalysemodul aus der Mehrzahl von Signalanalysemodulen enthält, und die Verzögerungsschaltung (116) ein Referenzsignal (RS) gemäß dem Messsignal (SS) generiert.

10. Erfassungssystem gemäß Anspruch 9, wobei das in dem Sensor enthaltene Signalanalysemodul des Weiteren enthält:

   eine mit der Verzögerungsschaltung (116) und einer aus der Mehrzahl von Empfangsantennen gekoppelte Mischerschaltung (132) zum Mischen des reflektierten Signals (RFS) und des Referenzsignals (RS), sodass es ein Mischsignal (MS) ist;
   eine mit der Mischerschaltung (132) gekoppelte Signalverstärkungsschaltung (134) zum Verstärken des Mischsignals (MS) zu einem verstärkten Mischsignal (AMS);
   einen mit der Signalverstärkungsschaltung (134) gekoppelte Bandpassfilterungsschaltung (136) zur Durchführung einer Filterungsoperation am verstärkten Mischsignal (AMS) zum Generieren eines gefilterten Signals (FS); und
   eine mit der Bandpassfilterungsschaltung (136) gekoppelte Abtastschaltung (138) zur Durchführung einer Abtastoperation an dem gefilterten Signal (FS) zur Erzielung eines Objektaktivstatussignals.

11. Erfassungssystem gemäß Anspruch 1, wobei das Erfassungssystem durch einen Chip implementiert ist, und in den Chip die mindestens eine Sendeantenne, die Mehrzahl von Empfangsantennen und eine integrierte Schaltung mit der einen oder den mehreren Überschwingungs- und Unterschwingungswellen generierenden Schaltungen und der Mehrzahl von Sensoren integriert sind.

12. Erfassungssystem gemäß Anspruch 1, wobei das Erfassungssystem des Weiteren eine Signalverarbeitungseinrichtung enthält, und die Signalverarbeitungseinrichtung des Weiteren enthält:

ein Drahtlosmodul, das mit der Mehrzahl von Sensoren kommuniziert; und

einen Mikrocontroller, der eine Berechnungseinheit anwendet, um einen oder mehrere Drücke des Zielobjekts zu berechnen.

13. Erfassungssystem gemäß Anspruch 12, wobei die Berechnungseinheit einen Algorithmus verwendet, um einen oder mehrere Blutdrücke zu berechnen, und der Algorithmus eine Beziehungsformel zwischen dem einen oder den mehreren Drücken und einer Pulswellengeschwindigkeit (PWV) enthält, und die PWV ein Maß für zwei Messpunkte (631, 632) des Zielobjekts ist.

14. Erfassungssystem gemäß Anspruch 13, wobei die PWV ausgedrückt wird als eine Funktion des Abstands (D) zwischen zwei verschiedenen Empfangsantennen (RX1, RX2) aus der Mehrzahl von Antennen und einer Zeitdifferenz ($\Delta$T) von zwei Signalen, die durch zwei verschiedene Sensoren aus der Mehrzahl von Sensoren jeweils empfangen werden.

15. Erfassungssystem gemäß Anspruch 14, wobei die Zeitdifferenz ($\Delta$T) eine Zeitspanne ist, die durch zwei verschiedene Generierungszeiten von zwei verschiedenen Pulsspitzen gewonnen wird, die durch die zwei verschiedenen Antennen (RX1, RX2) empfangen werden.

16. Erfassungsverfahren für physiologische Messungen, umfassend:

an einer Sendeseite (210), das Generieren, mit jeder aus einer Mehrzahl von Überschwingungs- und Unterschwingungswellen generierenden Schaltungen, eines Messsignals (SS) gemäß einem PWM-Signal (Pulse Width Modulation, Pulsweitenmodulation) (PS), und das Emittieren, mit jeder der mindestens einen Sendeantenne, des Messsignals (SS) zu einem Zielobjekt, wobei das Messsignal (SS) ein Merkmal einer Pulswelle hat und Überschwingungs- und Unterschwingungswellen ebenfalls auf der Pulswelle sind;
an einer Sendeseite (220), das Empfangen, mit jeder aus einer Mehrzahl von Empfangsantennen, ein reflektiertes Signal (RFS), das durch das Zielobjekt reflektiert wird;
das Generieren, durch eine Mehrzahl von Signalanalysemodulen, die jeweils in einer Mehrzahl von Sensoren enthalten sind, einer Mehrzahl von Objektaktivstatussignalen (231~23n), indem das reflektierte Signal (RFS) von jeder Empfangsantenne analysiert wird; und
das gleichzeitige oder ungleichzeitige Senden der Mehrzahl von Objektaktivstatussignalen (231~23n) zu einer digitalen Signalverarbeitungseinrichtung.

17. Erfassungsverfahren gemäß Anspruch 16, wobei das Messsignal (SS) generiert wird, indem das PWM-Signal (PS) gemäß einem Taktsignal (TS) geniert und das PWM-Signal mithilfe einer digitalen Signalverarbeitung moduliert wird, sodass es das Messsignal (SS) mit den Überschwingungs- und Unterschwingungspulsen ist.

18. Erfassungsverfahren gemäß Anspruch 16, wobei ein asymmetrisches kammförmiges Antennenmodul oder verschiedene Variationen im Hinblick auf das asymmetrische kammförmige Antennenmodul als die mindestens eine Sendeantenne eines Sendeantennenmoduls (322) und die Mehrzahl von Empfangsantennen an der Empfangsseite (220) verwendet werden.

19. Erfassungsverfahren gemäß Anspruch 16, wobei das Erfassungsverfahren des Weiteren eine Berechnung einer Druckmessung des Zielobjekts durchführt und die Berechnung der Druckmessung einschließt:

das Berechnen einer Pulszeitdifferenz einer ersten Pulsspitze und einer zweiten Pulsspitze, wobei die erste Pulsspitze und die zweite Pulsspitze durch zwei verschiedene Empfangsantennen (RX1, RX2) aus der Mehrzahl von Empfangsantennen empfangen werden;
das Berechnen einer Pulswellengeschwindigkeit (PWV) durch Verwendung der Pulszeitdifferenz und eines Abstands (D) zwischen der ersten Pulsspitze und der zweiten Pulsspitze; und
das Berechnen jedes Drucks von einem oder mehreren Drücken, indem eine lineare Beziehung zwischen dem Druck und der Pulswellengeschwindigkeit ausgedrückt wird.

20. Erfassungsverfahren gemäß Anspruch 19, wobei die Druckmessung ausgewählt ist aus einer oder mehreren Kombinationen der Folgendem: eine Blutdruckmessung einer Mehrzahl von Hauptarterien eines Brustkorbs, eine Blutdruckmessung einer Mehrzahl von Karotisarterien eines Halses, eine Hirndruckmessung, eine Blutdruckmessung eines peripheren Gefäßes.

**Revendications**

1. Un système de détection pour mesures physiologiques, comprenant :

une extrémité de transmission (210) incluant un module de génération de signal de mesure (214) ayant un ou plusieurs circuits de génération d'ondes de suroscillation et de sous-oscillation, chaque circuit de génération d'ondes de suroscillation et de sous-oscillation générant un signal de mesure (SS) selon un signal à modulation de largeur d'impulsions (PWM) (PS), et un module d'antenne de transmission (322) ayant au moins une antenne de transmission, chaque antenne de transmission émettant le signal de mesure vers un objet cible, dans lequel le signal de mesure (SS) a une caractéristique d'une onde pulsée, et les ondes de suroscillation et de sous-oscillation sont également sur l'onde pulsée ; une extrémité de réception (220) ayant une pluralité d'antennes de réception, chaque antenne de réception recevant un signal réfléchi (RFS) réfléchi par l'objet cible ; et une pluralité de modules d'analyse de signaux inclus dans une pluralité de détecteurs pour générer une pluralité de signaux d'état actif d'objet (231~23n) en analysant le signal réfléchi (RFS) provenant de chaque antenne de réception, et ensuite transmettre la pluralité de signaux d'état actif d'objet (231~23n) simultanément ou non simultanément à un processeur de signaux numériques.

2. Le système de détection tel que revendiqué dans la revendication 1, dans lequel le module d'antenne de transmission (322) est implémenté par une antenne de transmission, et une pluralité de signaux réfléchis (RFS1~RFSn) reçus par la pluralité d'antennes de réception sont une pluralité de signaux de mesure qui sont transmis par ladite une antenne de transmission à l'objet cible et réfléchis par l'objet cible.

3. Le système de détection tel que revendiqué dans la revendication 1, dans lequel le module d'antenne de transmission (322) est implémenté par une pluralité d'antennes de transmission, et une pluralité de signaux réfléchis (RFS1~RFSn) reçus par la pluralité d'antennes de réception sont une pluralité de signaux de mesure (SS1~SSn) qui sont transmis respectivement par ladite pluralité d'antennes de transmission à l'objet cible et réfléchis par l'objet cible.

4. Le système de détection tel que revendiqué dans la revendication 1, dans lequel le module de génération de signal de mesure (214) est implémenté par une pluralité de circuits de génération d'ondes de suroscillation et de sous-oscillation, chaque circuit de génération d'ondes de suroscillation et de sous-oscillation étant couplé à un détecteur associé parmi la pluralité de détecteurs pour générer le signal de mesure (SS) utilisé par le détecteur associé, respectivement.

5. Le système de détection tel que revendiqué dans la revendication 1, dans lequel le module de génération de signal de mesure (214) est implémenté par un circuit de génération d'ondes de suroscillation et de sous-oscillation couplé à la pluralité de détecteurs pour générer une pluralité de signaux de mesure selon une série de signaux à PWM entrés, et ensuite le module d'antenne de transmission (322) transmet la pluralité de signaux de mesure à l'objet cible.

6. Le système de détection tel que revendiqué dans la revendication 1, dans lequel l'extrémité de transmission (210) inclut en outre un module de circuit à PWM (412) couplé au module de génération de signal de mesure (214) pour générer le signal à PWM (PS) selon un signal d'horloge (TS).

7. Le système de détection tel que revendiqué dans la revendication 1, dans lequel le système de détection est en outre configuré en une zone de circuit (710) contenant le module de génération de signal de mesure (214) et la pluralité de détecteurs, et une pluralité de zones d'antenne contenant la pluralité d'antennes de réception et le module d'antenne de transmission (322) entouré par la pluralité d'antennes de réception.

8. Le système de détection tel que revendiqué dans la revendication 1, dans lequel pour l'au moins une antenne de transmission du module d'antenne de transmission (322) et la pluralité d'antennes de réception au niveau de l'extrémité de réception (220) ont respectivement une structure en forme de peigne.

9. Le système de détection tel que revendiqué dans la revendication 1, dans lequel chaque détecteur parmi la pluralité de détecteurs inclut en outre un circuit à retard (116) et un module d'analyse de signaux parmi la pluralité de modules d'analyse de signaux, et le circuit à retard (116) génère un signal de référence (RS) selon le signal de mesure (SS).

10. Le signal de détection tel que revendiqué dans la revendication 9, dans lequel le module d'analyse de signaux inclus dans le détecteur inclut en outre :

un circuit mixeur (132) couplé au circuit à retard (116) et à une antenne parmi la pluralité d'antennes de réception, et le fait de mixer le signal réfléchi (RFS) et le signal de référence (RS) pour en faire un signal de mixage (MS) ;

un circuit d'amplification de signal (134) couplé au circuit mixeur (132), et le fait d'amplifier le signal de mixage (MS) pour en faire un signal de mixage amplifié (AMS) ;

un circuit de filtrage passe-bande (136) couplé au circuit d'amplification de signal (134) et le fait de réaliser une opération de filtrage sur le signal de mixage amplifié (AMS) pour générer un signal filtré (FS) ; et

un circuit d'échantillonnage (138) couplé au circuit de filtrage passe-bande (136) et le fait de réaliser une opération d'échantillonnage sur le signal filtré (FS) pour obtenir un signal d'état actif d'objet.

**11.** Le système de détection tel que revendiqué dans la revendication 1, dans lequel le système de détection est implémenté par une puce, et la puce intègre l'au moins une antenne de transmission, la pluralité d'antennes de réception, et un circuit intégré ayant le ou les circuits de génération d'ondes de suroscillation et de sous-oscillation et la pluralité de détecteurs.

**12.** Le système de détection tel que revendiqué dans la revendication 1, dans lequel le système de détection inclut en outre un dispositif de traitement de signaux, et le dispositif de traitement de signaux inclut en outre :

un module sans fil qui communique avec la pluralité de détecteurs ; et

un microcontrôleur qui applique une unité de calcul pour calculer une ou plusieurs pressions de l'objet cible.

**13.** Le système de détection tel que revendiqué dans la revendication 12, dans lequel l'unité de calcul utilise un algorithme pour calculer une ou plusieurs pressions artérielles, et l'algorithme inclut une formule de relation entre le ou les pressions et une vitesse d'onde pulsée (PWV), et la PWV est une mesure pour deux points de mesure (631, 632) de l'objet cible.

**14.** Le système de détection tel que revendiqué dans la revendication 13, dans lequel la PWV est exprimée comme une fonction d'une distance (D) entre deux antennes de réception différentes (RX1, RX2) parmi la pluralité d'antennes, et une différence de durée (ΔT) de deux signaux reçus par deux détecteurs différents parmi la pluralité de détecteurs, respectivement.

**15.** Le système de détection tel que revendiqué dans la revendication 14, dans lequel la différence de durée (ΔT) est un intervalle de temps obtenu à travers deux moments de génération différents de deux crêtes d'impulsion différentes reçues par les deux antennes différentes (RX1, RX2).

**16.** Un procédé de détection pour mesures physiologiques, comprenant :

au niveau d'une extrémité de transmission (210), le fait de générer, avec chaque circuit parmi une pluralité de circuits de génération d'ondes de suroscillation et de sous-oscillation, un signal de mesure (SS) selon un signal à modulation de largeur d'impulsions (PWM) (PS), et le fait d'émettre, avec chaque antenne parmi au moins une antenne de transmission, le signal de mesure (SS) vers un objet cible, dans lequel le signal de mesure (SS) a une caractéristique d'une onde pulsée, et les ondes de suroscillation et de sous-oscillation sont également sur l'onde pulsée ;

au niveau d'une extrémité de réception (220), le fait de recevoir, avec chaque antenne parmi une pluralité d'antennes de réception, un signal réfléchi (RFS) réfléchi par l'objet cible ;

le fait de générer, par une pluralité de modules d'analyse de signaux respectivement inclus dans une pluralité de détecteurs, une pluralité de signaux d'état actif d'objet (231~23n) en analysant le signal réfléchi (RFS) provenant de chaque antenne de réception ; et

le fait de transmettre la pluralité de signaux d'état actif d'objet (231~23n) simultanément ou non simultanément à un dispositif de traitement de signaux numériques.

**17.** Le procédé de détection tel que revendiqué dans la revendication 16, dans lequel le signal de mesure (SS) est généré en générant le signal à PWM (PS) selon un signal d'horloge (TS), et en modulant le signal à PWM (PS) pour en faire le signal de mesure (SS) avec les impulsions de suroscillation et de sous-oscillation au moyen d'un traitement de signaux numériques.

**18.** Le procédé de détection tel que revendiqué dans la revendication 16, dans lequel un module d'antenne en forme de peigne asymétrique, ou diverses variations concernant le module d'antenne en forme de peigne asymétrique est utilisé comme au moins une antenne de transmission d'un module d'antenne de transmission (322) et la pluralité d'antennes de réception au niveau de l'extrémité de réception (220).

19. Le procédé de détection tel que revendiqué dans la revendication 16, le procédé de détection réalisant en outre un calcul d'une mesure de pression de l'objet cible, et le calcul de la mesure de pression inclut :

le fait de calculer une différence de durée d'impulsion entre une première crête d'impulsion et une deuxième crête d'impulsion, dans lequel la première crête d'impulsion et la deuxième crête d'impulsion sont reçues par deux antennes de réception différentes (RX1, RX2) parmi la pluralité d'antennes de réception ;
le fait de calculer une vitesse d'onde pulsée (PWV) en utilisant la différence de durée d'impulsion et une distance (D) entre la première crête d'impulsion et la deuxième crête d'impulsion ; et
le fait de calculer chaque pression parmi la ou les pressions, en exprimant une relation linéaire entre la pression et la vitesse d'onde pulsée.

20. Le procédé de détection tel que revendiqué dans la revendication 19, dans lequel la mesure de pression est choisie parmi une ou plusieurs combinaisons parmi une mesure de la pression artérielle d'une pluralité d'artères principales d'une poitrine, une mesure de la pression artérielle d'une pluralité d'artères carotides d'un cou, une mesure de la pression cérébrale, une mesure de la pression artérielle d'un vasculaire périphérique.

**FIG. 1A**

**FIG. 1B**

**FIG. 1C**

**FIG. 2**

**FIG. 3**

**FIG. 4**

**FIG. 5**

**FIG. 6**

701      703      702

| receiving antenna area | transmitting antenna area | receiving antenna area |
|---|---|---|

$\longleftarrow$  D  $\longrightarrow$

circuit area

sensing system

710

# FIG. 7

RX1 ┃ ┃ TX ┃ RX2    RX1 ┃ ┃ TX ┃ RX2    RX1 ┃ ┃ TX ┃ RX2    RX1 ┃ ┃ TX ┃ RX2

**FIG . 8A**          **FIG . 8B**          **FIG . 8C**          **FIG . 8D**

**FIG. 9**

**FIG. 10**

**FIG . 11**

Related to arterial stiffness

**FIG. 12**

**FIG. 13**

Transmission End

generating, with each of a plurality of overshoot and undershoot wave generating circuit, a measuring signal according to a PWM signal — 1410

emitting, with each of at least one transmitting antenna, the measuring signal to a target object, wherein the measuring signal has a characteristics of a pulse wave, and overshoot and undershoot waves are also on the pulse wave — 1412

Receiving End

receiving, with each of a plurality of receiving antennae, a reflected signal reflected by the target object — 1420

generating, by a plurality of signal analyzing modules included in a plurality sensors, a plurality of object active state signals by analyzing the reflected signal from the each receiving antenna — 1430

transmitting the plurality of object active state signals simultaneously or non-simultaneously to a DSP device — 1432

## FIG. 14